Europäisches Patentamt

European Patent Office

Office européen des brevets

(19)

(11) Veröffentlichungsnummer: **0 006 460**
**B1**

(12) **EUROPÄISCHE PATENTSCHRIFT**

(21) Anmeldenummer: **79101649.6**

(22) Anmeldetag: **30.05.79**

(51) Int. Cl.³: **C 07 C 31/20, C 07 C 29/14**

(54) Verfahren zur Gewinnung von reinem Neopentylglykol

(30) Priorität: **24.06.78 DE 2827795**

(43) Veröffentlichungstag der Anmeldung:
**09.01.80 Patentblatt 80/01**

(45) Bekanntmachung des Hinweises auf die Patenterteilung:
**12.11.80 Patentblatt 80/23**

(84) Benannte Vertragsstaaten:
**BE DE FR GB IT NL**

(56) Entgegenhaltungen:
**DE - A - 1 804 984**
**US - H - T892 025**

(73) Patentinhaber: **CHEMISCHE WERKE HÜLS AG**
**Postfach 1320**
**D - 4370 Marl 1 (DE)**

(72) Erfinder: **zur Hausen, Manfred, Dr.**
**Hoechster Strasse 1**
**D-4370 Marl (DE)**
**Kaufhold, Manfred, Dr.**
**Bitterfelder Strasse 7a**
**D-4370 Marl (DE)**
**Lange, Erhard, Dr.**
**Riegestrasse 14**
**D-4370 Marl (DE)**

Courier Press, Leamington Spa, England.

# Verfahren zur Gewinnung von reinem Neopentylglykol

Die Herstellung von Neopentylglykol (abgekürzt; NPG) erfolgt in bekannter Weise durch Umsetzung von Isobutyraldehyd mit Formaldehyd in Gegenwart von basischen Verbindungen wie Alkalilauge, Alkalicarbonaten, tertiären Aminen u. a. als Katalysatoren. Während die Synthese einfach durchgeführt werden kann, ist die Reinigung des hierbei entstandenen, durch Ester verunreinigten NPG technisch aufwendig. Es gibt zwar Verfahren, bei denen ein relativ reines Rohprodukt entsteht, wie z.B. bei Verfahren, bei denen tertiäre Amine als Katalysatoren eingesetzt werden oder die mit Zusatz von wasserlöslichen Alkoholen in homogener Phase arbeiten (vgl. "Ullmanns Enzyklopädie der technischen Chemie", Band 7 (1973), Seite 228; exakte Analysenwerte werden nicht veröffentlicht). Aber bei diesen Arbeitsweisen wird der Vorteil größerer Reinheit des Rohproduktes erkauft mit einem erheblichen Mehraufwand bei der Synthese: Denn die tertiären Aminkatalysatoren müssen in zwei zusätzlichen Destillationsschritten—der erste direkt nach der Synthese und der zweite nach der Hydrierung (bei der die Formiate zerlegt werden)—zurückgewonnen werden. Zudem ist eine besonders sorgfältige destillative Reinigung der Reaktionsprodukte erforderlich, um deren Stickstoffgehalt so gering wie nur möglich zu halten.

Setzt man in die Synthese einen wasserlöslichen Alkohol, z. B. Methanol, ein, so macht dieser die Abtrennung des Abwassers, das aus der wäßrigen Formalinlösung und der Alkalilauge bzw. Alkalicarbonatlösung stammt, unmöglich. Deshalb wird in zusätzlichen Verfahrensstufen Methanol und nicht umgesetzter Isobutyraldehyd abdestilliert, der Destillationsrückstand mit n-Dibutylether extrahiert, der Extrakt mit Wasser gewaschen, hydriert und danach der Ether destillativ abgetrennt.

Die US—PS 3 939 216 lehrt zwar, daß die Umsetzung von Isobutyraldehyd mit Formaldehyd in Gegenwart von wäßriger Sodalösung mit sehr geringem Aufwand problemlos durchführbar ist. Das bei dem hier beschriebenen Verfahren anfallende Rohprodukt enthält aber 10 bis 15% Ester, und zwar nicht nur den vom NPG durch Destillation leicht abtrennbaren Hydroxipivalinsäure-NPG-Ester, sondern, was nicht offenbart wird, auch den Mono-i-Buttersäure NPG-Ester.

Dieser Ester kann auf destillativem Wege nicht vom NPG mit sinnvollem technischem Aufwand getrennt werden. Da er bisher auch nur unter extremen Bedingungen, bei denen NPG zum Teil zersetzt wird, zum NPG und Isobutanol hydriert werden kann, ist er die wesentliche Ursache für die Reinigungs- bzw. Herstellungsprobleme des NPG.

Bei dem in dieser Patentschrift beschriebenen Verfahren wird das Entfernen der Ester auf folgendem Wege erreicht:

Nach der Hydrierung werden die Ester mit Natronlauge verseift und das Produkt anschließend einer Wasserdampfdestillation im Vakuum unterworfen. Das geschieht unter möglichst milden Bedingungen bei Temperaturen unter 140°C und kurzer Verweilzeit mit Hilfe eines Dünnschichtverdampfers, um Verluste an NPG weitgehend zu vermeiden. Damit die im Dünnschichtverdampfer anfallenden Salze fließfähig bleiben, darf ihr Gehalt an NPG einen bestimmten Wert nicht unterschreiten. Andernfalls treten Verstopfungen im Dünnschichtverdampfer auf, die nur nach Abstellen des Apparates durch Spülen mit Wasser beseitigt werden können. Durch die Abstellungen wird die Produktionskapazität des Dünnschichtverdampfers um 10 bis 15% gemindert. Deshalb läßt man eine gewisse Menge an NPG in den Abfallsalzen und arbeitet sie in einer besonderen Verfahrensstufe auf. Am Kopf des Dünnschichtverdampfers fällt eine verdünnte, wäßrige NPG-Lösung an, von der das Wasser in einer viel Energie verbrauchenden Destillationsstufe abgetrennt wird.

Weil diese Arbeitsweise sehr aufwendig ist, hat es nicht an Versuchen gefehlt, esterhaltiges NPG direkt zu reinem NPG zu hydrieren. Die DE—OS 18 04 984 (= GP—PS 1 219 162) schlägt als Hydrierkatalysatoren mit Barium aktivierte Kupferchromitkatalysatoren und Temperaturen von 175 bis 220°C vor.

Es wird gelehrt, daß der Hydroxipivalinaldehyd leicht bei Temperaturen unter 160°C zum NPG hydriert werden kann, daß zur Reduktion der als Verunreinigungen vorhandenen Ester aber Temperaturen von 200 bis 210°C benötigt werden. Hierbei wurde ein Produkt eingesetzt, das aufgrund seiner Herstellung, die mit Methanolzusatz durchgeführt wurde (s. o.), wenig Ester enthielt. Trotzdem sind diese hohen Temperaturen erforderlich, um Estergehalte von 0,1% zu erhalten. Die relativ niedrigen und schwankenden Ausbeuten von weniger als 80% zeigen, daß bei den hohen Hydriertemperaturen NPG zum Teil zersetzt wurde.

Die DE—AS 20 54 601 (= US—PS 4 094 914) stellt allgemein fest, daß die Hydrierung von Hydroxialdehyden in flüssiger Phase, bedingt durch lange Verweilzeiten, die zur völligen Reduktion benötigt werden, von der Bildung unerwünschter Nebenprodukte begleitet ist, wodurch Ausbeute und Reinheit der Diole beeinträchtigt werden. Der Effekt wird durch die aus der Synthese stammenden Restalkaligehalte noch verstärkt. Deshalb wird in dieser Schrift vorgeschlagen, in der Gasphase zu hydrieren. Hier wird mit einem Nickel enthaltenden Katalysator bei 128°C eine Selektivität zwischen 98 und 99% erreicht. Das eingesetzte Produkt enthielt aber nicht den Mono-iso-Buttersäureester des NPG (abgekürzt: MB—NPG). Es wurde also nicht geoffenbart, ob mit diesem Verfahren das oben geschilderte Hauptproblem bei der NPG-Herstellung gelöst werden kann. Ein Nachteil des Verfahrens ist zudem der hohe Energieverbrauch, weil das eingesetzte Produkt und zusätzlich noch das benötigte Lösemittel verdampft werden müssen.

Alle bekannten Verfahren zeigen somit erhebliche Nachteile auf, indem sie entweder durch besondere, aufwendige Maßnahmen in der Synthese die Bildung von MB—NPG vermeiden, oder diesen Ester durch alkalische Verseifung entfernen und dann durch aufwendige technische Einrichtungen den schädlichen Einfluß der Natriumsalze so gering wie nur möglich halten müssen.

Damit stellt sich die Aufgabe, ein Verfahren zu finden, mit dem bei minimalem technischem Aufwand ein NPG mit einem durch gaschromatographische Analyse ermittelten Reinheitsgrad von über 98% gewonnen werden kann.

Diese Aufgabe wird erfindungsgemäß entsprechend den Angaben der Patentansprüche gelöst.

Es wurde überraschenderweise gefunden, daß man entgegen der oben erwähnten Ansicht durch Hydrierung in flüssiger Phase ohne Bildung von Nebenprodukten und ohne wesentliche Zersetzung von NPG reines NPG aus esterhaltigem, MB—NPG und geringe Mengen an Wasser enthaltendem rohem Hydroxipivalinaldehyd gewinnen kann, indem man in zwei Stufen hydriert, und zwar zuerst bei tiefen Temperaturen und anschließend bei höheren Temperaturen. Als Katalysatoren werden bei dem erfindungsgemäßen Verfahren mit Barium aktivierte Kupferchromitkatalysatoren eingesetzt, wie sie z. B. in der US—PS 3 340 312 vorgeschlagen werden.

Diese mit Barium aktivierten Kupferchromitkatalysatoren sind bekannt (US-PPS 2 137 407; 2 091 800; 2 782 243 und 2 544 771 und Adkins et al J. Am. Chem. Soc. 53,1 091 (1931); J. Am. Chem. Soc. 53, 1 095 (1931); J. Am. Chem. Soc. 54, 1 145 (1932); Connor et al, Am. Chem. Soc. 54, 1 138 (1932); Adkins et al, J. Am. Chem. Soc. 72, 2 626 (1950)) und haben beispielsweise folgende Zusammensetzung: 33 bis 35% CuO, 38% $CrO_3$, 10% BaO sowie $SiO_2$ als Bindemittel. Der Katalysator kann auf einen Träger aufgetragen sein. Es ist wesentlich, daß dieser mit Barium aktivierte Kupferchromitkatalysator in beiden Stufen eingesetzt wird. Die Hydrierung in der zweiten Stufe verläuft nur dann zufriedenstellend, wenn das Ausgangsprodukt auch in der ersten Stufe erfindungsgemäß mit einem mit Barium aktivierten Kupferchromitkatalysator vorhydriert wird (s. Vergleichsbeispiel 3).

Das Hydrierproblem wird erfindungsgemäß dadurch gelöst, daß der rohe MB—NPG und geringe Mengen an Wasser enthaltende Hydroxipivalinaldehyd in der ersten Stufe über einen mit Barium aktivierten Kupferchromitkatalysator bei Temperaturen von 120 bis 160°C, vorzugsweise von 140 bis 150°C, und 200 bis 300 bar Wasserstoffdruck und anschließend in der zweiten Stufe über einen ähnlichen oder gleichen Kupferchromitkatalysator bei Temperaturen von 170 bis 200°C, vorzugsweise von 175 bis 190°C, und 200 bis 300 bar Wasserstoffdruck hydriert wird. Die zweite Stufe führt nur dann zu geten Ergebnissen, wenn bei der ersten diese Bedingungen sowie Belastungen von 0,05 bis 1,0, vorsugsweise von 0,1 bis 0,5 l rohen Hydroxypivalinaldehyd/1 Katalysator.h eingehalten werden. Dieser Belastungsbereich ist auch in der zweiten Stufe erforderlich. Es hat sich überraschenderweise gezeigt, daß Produkte, die bei zu hoher Temperatur oder über einen ungeeigneten Katalysator in der ersten Stufe hydriert wurden, in der zweiten Stufe nur unter Bildung von Nebenprodukten und unter merklicher Zersetzung zu einem NPG hydriert werden, dessen Reinheitsgrad unter 98% liegt. Dieses stark verunreinigte NPG läßt sich destillativ nicht weiter reinigen, obwohl der Gehalt an MB—NPG mit Werten von 0,8 bis 0,5% relativ gering ist (s. Vergleichsbeispiel 3).

Weiter wurde gefunden, daß ein gewisser Gehalt an Wasser im rohen Hydroxipivalinaldehyd die Zersetzung von NPG während der Hydrierung stark zurückdrängt. Besonders vorteilhaft ist ein Gehalt von 3 bis 8% der sich durch Sättigung der organischen Phase mit Wasser einstellt und der bei den erfindungsgemäßen Beispielen 4,85% und 6,12% beträgt. Reines, beispielsweise in iso-Butanol gelöstes, NPG ist unter den Hydrierbedingungen überraschenderweise nur in Gegenwart von Wasser stabil. Wassergehalte weit über 8% vermindern, wie bekannt, die Lebensdauer der Hydrierkatalysatoren.

Das erfindungsgemäße Verfahren wird anhand der folgenden Beispiele näher erläutert. Die Erfindung bezieht sich nur auf die Hydrierung des rohen Hydroxipivalinaldehyds und nicht auf dessen Herstellung, die im folgenden nur als eine von vielen Möglichkeiten beispielhaft mitaufgeführt wird.

Beispiel 1.

*Herstellung von rohem Hydroxipivalinaldehyd*

Die Unsetzung von iso-Butyraldehyd mit Formaldehyd führt man in einer Aldolisierungsapparatur, die ein effecktives Volumen von 10 1 besitzt, durch. Man setzt pro Stunde 16 1 iso-Butyraldehyd und 8 1 Formalin (29prozentig) ein. Der pH-Wert wird durch Zugabe von 25prozentiger Natronlauge auf 10,0 eingestellt und automatisch geregelt. Die Reaktionstemperatur liegt bei 60°C. Der Austrag trennt sich in zwei Phasen, von denen die wäßrige verworfen wird. Die Ausbeute an rohem Hydroxipivalinaldehyd liegt bei 80% der Theorie (bezogen auf Formaldehyd). Das Produkt (= Produkt A) hat folgende Zusammensetzung (iso-Butanol-, Formaldehyd- und Wasserfrei gerechnet):

| | |
|---|---|
| Hydroxipivalinaldehyd | 65,6% |
| Neopentylglykol | 8,3% |
| Acetal aus iso-Butyraldehyd und NPG | 1,4% |
| mono-i-Buttersäureester des NPG | 6,5% |
| mono-Hydroxipivalinsäure-NPG-Ester | 7,2% |
| 2.2.4-Trimethylpentandiol-1,3 (= TMP) | 2,8% |
| TMP-mono-i-Butyrat | 0,2% |
| Summe sonstiger Verbindungen | 8,0% |

Der zusätzlich bestimmte Wassergehalt beträgt 4,85%.

Wird bei sonst unveränderten Bedingungen der pH-Wert auf 10,5 eingestellt, so erhöht sich die Ausbeute an rohem Hydroxipivalinaldehyd auf 87% der Theorie, bezogen auf eingesetzten Formaldehyd. Hinsichtlich der Hydrierung ist wichtig, daß der Gehalt an Estern zunimmt. Der Reaktionsaustrag (= Produkt B) hat folgende Zusammensetzung:

| | |
|---|---|
| Hydroxipivalinaldehyd | 50,8% |
| Neopentylglykol | 9,8% |
| Acetal aus iso-Butyraldehyd und NPG | 1,6% |
| mono-i-Buttersäureester des NPG | 9,1% |
| mono-Hydroxyipivalinsäure-NPG-Ester | 13,8% |
| 2.2.4-Trimethylpentandiol-1,3 (= TMP) | 3,4% |
| TMP-mono-i-Butyrat | 0,2% |
| Summe sonstiger Verbindungen | 11,3% |

Der mit einer anderen Analysenmethode erfaßte Wassergehalt liegt bei 6,12%.

### Beispiel 2.

*2.1: 1. Hydrierstufe*

Das Produkt A aus Beispiel 1 hydriert man in einem Hochdruckreaktor, der ein effektives Volumen von 28 1 (Länge 5 m, Durchmesser 90 mm) hat und mit 24 1 eines mit Barium aktivierten Kupferchromitkatalysators folgender Zusammensetzung: 33% CuO, 38% $CrO_3$, 10% BaO, Rest $SiO_2$ (1107 T der Fa. HARSHAW) gefüllt ist, bei 145°C und 280 bis 300 bar Wasserstoffdruck. Man setzt pro Stunde 7 1 des iso-Butyraldehyd enthaltenden Aldolisierungsproduktes ein, d. h., die Belastung beträgt 0,292 (1 Aldolisierungsprodukt / 1 Katalysator.h). Es wird eine Kreisgasmenge von 200 <—>/h und eine Zufuhr an Frischgas von 5 bis 6<—>/h eingestellt.

Man destilliert eine Probe des Hydrieraustrages (= Produkt C) zur genaueren Untersuchung bei 124 mbar und untersucht den zwischen 146 und 148°C siedenden Hauptlauf gaschromatographisch; er besteht aus:

| | |
|---|---|
| NPG | 96,2% |
| MB—NPG | 3,6% |
| 2.2.4-Trimethylpentandiol-1,3 (= TMP) | 0,2% |

Die Selektivität hinsichtlich der Reduktion des Aldehyd-Ester-Gemisches zum NPG beträgt 97,8%.

# 0 006 460

Produktverluste dirch Zersetzung sind nicht entstanden. Der Umsatz an Hydroxipivalinaldehyd liegt über 99%.

Beim Einsatz des Produktes B aus Beispiel 1 und nach analoger Durchführung der Hydrierung sowie der Analytik des Hydrieraustrages (= Produkt D) zeigt das Gaschromatogramm folgende Zusammensetzung:

| | |
|---|---|
| NPG | 93,7% |
| MB—NPG | 5,6% |
| 2.2.4-Trimethylpentandiol-1,3 (= TMP) | 0,3% |
| sonstige Verbindungen | 0,4% |

Die Selektivität hinsichtlich der Reduktion des Aldehyd-Ester-Gemisches zum NPG beträgt 96,5%. Eine Zersetzung der Produkte während der Hydrierung ist nicht meßbar. Der Umsatz an Hydroxipivalinaldehyd liegt über 99%.

*2.2: 2. Hydrierstufe*

Das Produkt C (also vorhydriertes Produkt A) wird in gleicher Weise wie unter 2.1 beschrieben, jedoch bei erhöhten Temperaturen, hydriert. Als Hydriertemperaturen wurden 170, 180, 190 und 200°C gewählt. Die Ergebnisse zeigt folgende Tabelle:

TABELLE 1

| Hydrier-temperatur °C | Gaschromatographische Analyse des Destillats * (%) | | | | Esterumsatz der 2. Stufe ** (%) | Ausbeute *** (%) |
|---|---|---|---|---|---|---|
| | NPG | MB–NPG | TMP | Restliche Verbindungen | | |
| 170 | 98,2 | 1,2 | 0,2 | 0,4 | 66,7 | 99,9 |
| 180 | 99,3 | 0,5 | 0,2 | 0,0 | 86,1 | 99,8 |
| 190 | 99,4 | 0,5 | 0,1 | 0,0 | 86,1 | 97,5 |
| 200 | 99,6 | 0,4 | 0,0 | 0,0 | 88,9 | 71,2 |

\* Eine Probe des Hydrieraustrags wird, wie unter 2.1 beschrieben, destilliert, um eine exakte gaschromatographische Analyse durchführen zu können.

\*\* Unter Esterumsatz der zweiten Stufe wird folgendes verstanden : 3,6 %MB–NPG im Ausgangsprodukt werden zu 1,2 % im Endprodukt der zweiten Stufe reduziert, d. h., 2,4 % werden zum NPG hydriert, das sind 66,7 % Umsatz.

\*\*\* Die Ausbeute bezieht sich auf destilliertes Produkt mit angegebenen Reinheit.

In gleicher Weise hydriert man das Produkt D (also vor-hydriertes Produkt B) bei denselben Temperaturen durch. Diese Ergebnisse sind in Tabelle 2 zusammengefaßt:

5

# 0 006 460

TABELLE 2

| Hydrier-temperatur °C | Gaschromatographische Analyse des Destillats * (%) | | | | Esterumsatz der 2. Stufe ** (%) | Ausbeute *** (%) |
|---|---|---|---|---|---|---|
| | NPG | MB–NPG | TMP | Restliche Verbindungen | | |
| 170 | 98,1 | 1,4 | 0,2 | 0,3 | 75,0 | 99,9 |
| 180 | 99,2 | 0,6 | 0,2 | 0,0 | 89,3 | 99,5 |
| 190 | 99,1 | 0,7 | 0,2 | 0.0 | 87,5 | 95,5 |
| 200 | 99,5 | 0,5 | 0,0 | 0,0 | 91,1 | 68,7 |

*, **, *** siehe oben.

Diese Ergebnisse zeigen, daß mit dem erfindungsgemäßen Verfahren die Reduktion des MB—NPG bei erheblich tieferen Temperaturen (nämlich um 180°C) als in der Literatur angegeben (200 bis 220°C, s. o.), gelingt, und daß bei diesen Temperaturen eine ausgezeichnete Ausbeute erzielt wird.

Ferner beweist der bei 200°C durchgeführte Hydrierversuch, daß trotz starker Zersetzung (geringere Ausbeute) Keine Nebenprodukte gebildet werden, die das destillierte NPG verunreinigen sondern im Gegenteil das reinste Produkt erhalten wird. Dieses Ergebnis steht im Gegensatz zu den Angeben der DE—AS 20 54 601 (s. o.), daß in flüssiger Phase eine Hydrierung des Hydroxipivalinaldehyds ohne Bildung unerwünschter Nebenprodukte, die die Reinheit des Diols vermindern, nicht möglich ist.

Vergleichsbeispiel 3.

Da in der Literatur häufig Nickelkontakte zur Hydrierung des Hydroxipivalinaldehyds vorgeschlagen werden, wird ein Vergleichsversuch mit einem Nickelkatalysator folgender chemischer Zusammensetzung durchgeführt:

Ni (als Formiat)    10 bis 11 %

Cu          ca.  3  %

Cr          ca. 0,2%

SiO$_2$       ca. 65  %

Trocknungsverlust    ca.  1,  %

Ausgangsprodukt ist das im Beispiel 1 beschriebene Produkt B. Der eingesetzte Hydrierreaktor (Länge 930 mm, Durchmesser 45 mm) hat ein effektives Volumen von 1,4 1 und ist mit 1 300 ml des o. g. Katalysators gefüllt. Es wird eine Abgasmenge von 400 1 und eine Menge as Einsatzprodukt von 100 ml pro Stunde (Belastung von 0,08) eingestellt. Der Wasserstoffdruck beträgt 300 bar.

Um unter möglichst schonenden Bedingungen zu hydrieren, beginnt man mit einer Temperatur von 120°C. Da der Hydrieraustrag eine Carbonylzahl von 2,7 aufweist, d. h., die Aldehyde sind nicht vollständig hydriert, steigert man die Temperatur langsam. Selbst bei 160°C liegt die Carbonylzahl jedoch noch über 2 und die Verseifungszahl der iso-Butanol-haltigen Lösung über 59.

Anschließend gibt man den Hydrieraustrag nochmal unter den gleichen Bedingungen über den Katalysator. Hierbei fällt die Carbonylzahl von ca. 2 auf 0,1 ab, aber die Verseifungszahl wird nur geringfügig kleiner (von 59 auf 54).

Dieses zweimal hydrierte Produkt untersucht man bei einer Probedestillation näher. Bei der De-

6

stillation fällt ein Destillationsrückstand in mehr als der doppelten Menge (genauer Faktor: 2,10) gegenüber der Hydrierung mit dem im Beispiel 2 eingesetzten Katalysator (s. Beispiel 2.1) an, d. h., während der Hydrierung werden hochsiedende Produkte gebildet.

Eine Nachhydrierung bei 180°C über den im Beispiel 2 eingestzten Katalysator, in der im Vergleichsbeispiel 4 beschriebenen Weise, führt nur zu einer Verringerung der Verseifungszahl von 54 auf 33. Der Hydrieraustrag dieses Versuchs erbringt bei erneuter Nachhydrierung unter gleichen Bedingungen (180°C, im Beispiel 2 eingesetzter Katalysator) eine Verkleinerung der Verseifungszahl von 33 auf 18. Eine Verseifungszahl von 18 entspräche einem Estergehalt (wenn man einfachheitshalber alle Ester als MB—NPG in Rechnung setzt) von mindestens 13,9%, bezogen auf Feststoff. Dieser Wert ist also viel zu hoch.

Hydriert man in der ersten Stufe mit dem im Beispiel 2 eingesetzten mit Barium aktivierten Kupferchromitkatalysator bei Temperaturen über 160°C, beispielsweise bei 180°C, so erhält man zu geringe Ausbeuten von weniger als 90% sowie in der zweiten Stufe einen höheren Anteil an Nebenprodukten. Der Reinheitsgrad des erhaltenen NPG liegt unter 98%.

Diese Ergebnisse zeigen sehr deutlich, daß nur dann die Hydrierung in der zweiten Stufe zufriedenstellend durchführbar ist, wenn das Ausgangsprodukt in der ersten Stufe erfindungsgemäß vorhydriert wird.

### Vergleichsbeispiel 4. (zur 2. Stufe)

Um die Hydrierbarkeit des weitgehend wasserfreien MB—NPG zu untersuchen, stellt man durch partielle Veresterung von reinem NPG mit i-Buttersäure ein Substanzgemisch her, das nur NPG und MB—NPG enthält. Dieses Produkt verdünnt man mit soviel i-Butanol, daß eine 40prozentige Lösung entsteht. Ihr Estergehalt liegt bei 6,3% (= 15,9%, bezogen auf Festsubstanz) und der Wassergehalt bei nur 0,3%.

Die Lösung wird bei 170 und 180°C über den im Beispiel 2 eingesetzten Katalysator (1107 T der Fa. HARSHAW) bei 300 bar Wasserstoffdruck hydriert. Der Hydrierreaktor (Länge 60 cm, Durchmesser 16 mm) besitzt ein effektives Volumen von 110 ml und ist mit 100 ml des o. g. Katalysators gefüllt. Man stellt eine Abgasmenge von 200 1 und eine Menge an Einsatzprodukt von 20 ml pro Stunde ein.

Von den Hydrierausträgen werden Proben entnommen und diese destilliert, um exakte gaschromatographische Analysen durchführen zu können. Die Ergebnisse der beiden Versuche zeigt Tabelle 3:

### TABELLE 3

| Hydrier-temperatur °C | Gaschromatographische Analyse des Destillats (%) | | | Esterumsatz (%) | Ausbeute (%) |
|---|---|---|---|---|---|
| | NPG | MB—NPG | Restliche Verbindungen | | |
| 170 | 99,3 | 0,5 | 0,2 | 96,9 | 72,9 |
| 180 | 99,4 | 0,4 | 0,2 | 97,5 | 72,3 |

Diese Ergebnisse zeigen, daß bei einem weitgehend wasserfreien Einsatzprodukt selbst bei relativ niedrigen Temperaturen starke Zersetzung (geringere Ausbeute) stattfindet, daß aber trotzdem entgegen der in der DE—AS 20 54 061 geäußerten Meinung die Bildung von Nebenprodukten, die die Reinheit des NPG vermindert, nicht beobachtet wird. Der Esterumsatz ist überraschenderweise umso höher, je niedriger der Wassergehalt ist.

Führt man oben beschriebene Versuche unter gleichen Bedingungen, aber erfindungsgemäß unter Zusatz von z. B. 6% Wasser (bezogen auf die Lösung) durch, so erhöht sich die Ausbeute an NPG auf über 98%.

### Patentansprüche

1. Verfahren zur Gewinnung von Neopentylglykol mit einem Reinheitsgrad von über 98%, ermittelt durch gaschromatographische Analyse, durch katalytische Druckhydrierung eines rohen Hydroxipivalinaldehyds, der unter anderem den mono-iso-Buttersäureester des Xeopentylglykols als Verunreinigung sowie geringe Mengen an Wasser enthält, in flüssiger Phase über mit Barium aktivierte Kupferchromitkatalysatoren, dadurch gekennzeichnet, daß man rohen Hydroxipivalinaldehyd, der

7

geringe Mengen an Wasser enthält, in zwei Stufen unter Hochdruck hydriert, wobei man in der ersten Stufe Temperaturen von 120 bis 160°C und Belastungen von 0,05 bis 1,0 1 rohen Hydroxipiralin aldehyd/1 Katalysator. h und in der zweiten Stufe Temperaturen von 170 bis 200°C und Belastungen von 0,05 bis 1,01 vorhydrierter Hydroxipivalinaldehyd/1 Katalysator.h einstellt.

2. Verfahren nach Anspruch 1, dadurch gekennzeichnet, daß man in der ersten Stufe bei Temperaturen von 140 bis 150°C und in der zweiten Stufe bei Temperaturen von 175 bis 190°C hydriert.

3. Verfahren nach den Ansprüchen 1 und 2, dadurch gekennzeichnet, daß man in beiden Stufen bei Belastungen von 0,1 bis 0,5 1 Hydroxipivalinaldehyd/ 1 Katalysator.h hydriert.

4. Verfahren nach den Ansprüchen 1 bis 3, dadurch gekennzeichnet, daß man einen rohen Hydroxipivalinaldehyd mit einem Wassergehalt von 3 bis 8% einsetzt.

5. Verfahren nach den Ansprüchen 1 bis 4, dadurch gekennzeichnet, daß man in beiden Stufen bei einem Wasserstoffdruck von 200 bis 300 bar hydriert.

## Claims

1. A process for obtaining pure neopentyl glycol (NPG), having a degree of purity greater than 98%, as determined by gas-chromatographic analysis, by catalytic hydrogenation of a crude hydroxypivalaldehyde, which inter alia contains the mono-iso-butyric acid ester of NPG an an impurity, together with small amounts of water, under pressure in the liquid phase over barium-activated copper chromite catalysts, characterised in that crude hydroxypivalaldehyde, which contains small amounts of water, is hydrogenated in two stages under high pressure, the conditions set up in the first stage being temperatures of 120 to 160°C and throughputs of 0,05 to 1.0 litre of hydrogenation feed/litre of catalyst·hour and those set up in the second stage being temperatures of 170 to 200°C and throughputs of 0.05 to 1.0 litre of hydrogenation feed/litre of catalyst·hour.

2. A process according to claim 1, characterised in that the hydrogenation is carried out at temperatures of 140 to 150°C in the first stage and at temperatures of 175 to 190°C in the second stage.

3. A process according to claims 1 and 2, characterised in that in both stages the hydrogenation is carried out at throughputs of 0.1 to 0.5 litre of hydrogenation feed/litre of catalyst·hour.

4. A process according to claims 1 to 3, characterised in that a crude hydroxypivalaldehyde containing 3 to 8% of water is employed.

5. A process according to claims 1 to 4, characterised in that in both stages the hydrogenation is carried out under a hydrogen pressure of 200 to 300 bar.

## Revendications

1. Procédé pour obtenir, par hydrogénation catalytique sous pression d'un hydroxipivalinaldéhyde brut, contenant entre autres l'ester mono-isobutyrique du néopentylglycol (NPG) comme impureté ainsi que de faibles quantités d'eau, en phase liquide par l'intermédiaire de catalyseurs au chromite de cuivre activés avec du baryum, du NPG pur d'un degré de pureté de plus de 98%, déterminé par analyse par chromatographie en phase gazeuse, caractérisé par le fait qu'on hydrogène en deux étapes sous une pression élevée de l'hydroxipivalinaldéhyde brut, qui contient de faibles quantités d'eau, en ajustant dans la première étape des températures de 120 à 160°C et des charges de 0,05 à 1,01 de charge d'hydrogénation par litre de catalyseur solide fois heure, et dans la deuxième étape des températures de 170 à 200°C et des charges de 0.05 à 1,0 l de charge d'hydrogénation par litre de catalyseur solide fois heure.

2. Procédé suivant la revendication 1, caractérisé par le fait qu'on hydrogène dans la première épate à des températures de 140 à 150°C et dans la deuxième étape à des températures de 175 à 190°C.

3. Procédé suivant les revendications 1 et 2, caractérisé par le fait qu'on hydrogène dans les deux étapes à des charges de 0,1 à 0.5 l de charge d'hydrogénation par l de catalyseur solide fois heure.

4. Procédé suivant les revendications 1 à 3, caractérisé par le fait qu'on emploie un hydroxipivalinaldéhyde brut ayant une teneur en eau de 3 à 8%.

5. Procédé sulvant des revendications 1 à 4, caractérisé par le fait qu'on hydrogène dans les daux étapes avec une pression d'hydrogène de 200 à 300 bars.